# EUROPÄISCHE PATENTSCHRIFT

(11) **EP 0 478 727 B1**
(45) Veröffentlichungstag und Bekanntmachung des Hinweises auf die Patenterteilung: **21.08.1996**
(21) Anmeldenummer: 91906756.1
(22) Anmeldetag: 04.04.1991
(51) Int. Cl.: A61K 9/127

(54) **VERFAHREN ZUR HERSTELLUNG VON WIRKSTOFFHALTIGEN WÄSSRIGEN LIPOSOMENSUSPENSIONEN**
PROCESS FOR THE PREPARATION OF AQUEOUS LIPOSOME SUSPENSIONS CONTAINING ACTIVE SUBSTANCES
PROCEDE POUR LA FABRICATION DE SUSPENSIONS AQUEUSES DE LIPOSOMES CONTENANT DES MATIERES ACTIVES

(30) Priorität: 24.04.1990 DE 4013580
(43) Veröffentlichungstag der Anmeldung: 08.04.1992
(73) Patentinhaber: SCHERING AKTIENGESELLSCHAFT, D-13342 Berlin (DE)
(72) Erfinder: Rössling, Georg, Dr., D-1000 Berlin 28 (DE); SACHSE, Andreas, D-1000 Berlin 10 (DE); SCHRÖTER, Hans-Joachim, D-1000 Berlin 49 (DE); SPRENGER, Claudia, D-1000 Berlin 44 (DE); WAWRETSCHEK, Cornelia, D-1000 Berlin 22 (DE)
(86) Internationale Anmeldenummer: DE9100294
(87) Internationale Veröffentlichungsnummer: WO9116039

(56) Entgegenhaltungen:
- EP-A- 0 130 577
- EP-A- 0 349 429
- FR-A- 2 614 787
- GB-A- 2 140 822
- Derwent Datenbank-Lieferant WPIL, 1989, AN 89-181250(25), Derwent Publications Ltd, (London, GB), & JP-A-1117824 (TERUMO CORP.) 10. Mai 1989
- Proceedings of the Annual International Conference of the IEEE Engineering in Medicine and Biology Society, Teil 174: Cardiology and Imaging, 4.-7. November 1988, New Orleans, Louisiana, Band 10, IEEE, M. Capellier et al.: "Radiopaque liposomes for computed tomography opacification", Seiten 491-492

## Beschreibung

Die Erfindung betrifft ein Verfahren zur Herstellung von wirkstoffhaltigen wässrigen Liposomensuspensionen aus einer Lösung einer (eines) Liposomen bildenden Substanz(gemisches) (nachfolgend auch als Lipid oder Lipidgemisch bezeichnet) in einem niederen Alkohol mit maximal 3 Kohlenstoffatomen und einer wässrigen Phase wobei der Wirkstoff oder das Wirkstoffgemisch in der Lipidlösung und/oder der wässrigen Phase gelöst oder suspendiert ist, dadurch gekennzeichnet, daß man die wässrige Phase unter Homogenisieren in die alkoholische Lösung eintragt und den niederen Alkohol mittels Vakuumdestillation entfernt.

Bekanntlich sind Liposomen in sich geschlossene sphärische oder elliptische Lipidvesikel, die eine wässrige Phase einschließen. Je nach Anzahl der vorhandenen Lipiddoppelschichten unterscheidet man große und kleine unilamellare Liposomen (LUV und SUV) von multilamellaren Liposomen (MLV).

Die ersten Liposomen wurden durch Dispersion von Lipidfilmen in wässrigen Phasen hergestellt. Die so erhaltenen MLV-Dispersionen können durch Anwendung geeigneter Homogenisationsverfahren, wie z.B. Ultrabeschallung oder Hochdruckhomogenisation, in SUV überführt werden. MLV und SUV weisen jedoch nur ein geringes Einschlußvolumen (Volumen eingeschlossene wässrige Phase [Liter/mol Lipid] auf, so daß sie zum effektiven Einschluß hydrophiler Substanzen nicht geeignet sind.

LUV weisen demgegenüber ein deutlich vergrößertes Einschlußvolumen und damit eine erhöhte Einschlußkapazität (% des eingesetzten Arzneistoffs) auf.

Die bis zum heutigen Tage gängigsten Methoden zur Herstellung von LUV können unter dem Oberbegriff der "solvent evaporation" Methoden zusammengefaßt werden.

Die wohl bekannteste und im Hinblick auf die erhaltenen Einschlüsse beste Methode ist die REV (reverse phase evaporation) Methode von Papahadjopoulos (U.S Patent No. 4,235,871. Hierbei wird das Lipid beziehungsweise Lipidgemisch zunächst in einem mit Wasser nicht oder nur wenig mischbaren Lösungsmittel (in der Regel Diethylether oder Chloroform) gelöst. Nach Zugabe zur arzneistoffhaltigen wässrigen Phase wird das Gemisch dann durch Ultrabeschallung in eine W/O-Emulsion überführt. Aus dieser wird anschließend das Lösungsmittel am Rotationsverdampfer entfernt, wobei sich zunächst ein Gel ausbildet, welches durch Erhöhung des Vakuums oder Zugabe von Wasser in eine Liposomensuspension umgewandelt wird.

Eine spezielle Ausgestaltung dieses Verfahrens wird in der GB 2 140 822 A offenbart. Hierbei wird die Emulgierung ohne Ultraschallbehandlung durch intensive Durchmischung durchgeführt. Dieses Verfahren soll insbesondere dazu geeignet sein, DNA in Pflanzenzellen zu überführen.

Im Gegensatz hierzu wird bei der von Deamer und Bangham eingeführten "ether injection" Methode [Biochim. Biophys. Acta, 443 (1976) 629-634], daß ebenfalls in Diethylether gelöste Lipid in eine warme wässrige Lösung (50-60° C) bei Unterdruck injiziert. Die nach Filtration über ein 1,2 µm Filter erhaltenen Liposomen sind heterogen und weisen Größen zwischen 150-250 nm auf.

Die bei diesen Methoden verwendeten Lösungsmittel sind aus toxikologischen und sicherheitstechnischen Gründen kritisch zu beurteilen.

Verwendet man bei einem der "ether injection" Methode vorangegangenem Verfahren jedoch Ethanol als Lösungsmittel, so resultieren stark verdünnte SUV-Dispersionen, die durch Ultrafiltration aufkonzentriert werden [Biochim. Biophys. Acta, 298 (1973) 1015-1019]. Die mittlere Größe der so erhaltenen Liposomen liegt deutlich unter 50 nm und der MLV Anteil wird mit 6 % angegeben.

Eine Ausgestaltung dieses Verfahrens ist in der EP-A 0 253 619 beschrieben. Bei diesem Verfahren wird beispielsweise eine ethanolische Lipid- und Arzneimittel-Lösung, die insgesamt 10 % der Gesamtformulierung ausmachen unter Druck von 1 000 - 3 000 000 hPa in eine wässrige Phase, die durch einen Homogenisator agitiert wird, injiziert. Nach diesem Verfahren, daß sehr aufwendig ist, entstehen kleine unilammelare Liposomen. Zur Herstellung von Liposomensuspensionen, die hydrophile Wirkstoffe enthalten, ist dieses Verfahren ungeeignet.

Ein gänzlich anderes Verfahren ist in der EP 130 577 A2 beschrieben. Hierbei werden die Lipide in einem wasserlöslichen nicht-flüchtigem Lösungsmittel gemischt. Dazu wird eine, gegebenenfalls wirkstoffhaltige, wäßrige Lösung gegeben und die so erhaltene Mischung dispergiert, wobei sich die gewünschten Liposomen bilden. Dieses Verfahren besitzt den Nachteil, daß das nicht-flüchtige Lösungsmittel nicht abgetrennt werden kann und somit in den Liposomen verbleibt.

Erwahnenswert ist auch eine Methode zur Herstellung von Liposomen mit wasserloslichen Losungsmitteln, welche die Herstellung einer "monophase" beinhaltet (WO 85/00751). Diese einphasige Mischung wird in dar Regel aus 5 ml des lipidhaltigen Losungsmittels (z.B. Ethanol) und 0.2 ml der wässrigen Komponente hergestellt. Das Losungsmittel wird anschließend durch Einleiten eines Inertgases bei gleichzeitiger Beschallung der Mischung entfernt wobei ein Film entsteht. Dieser wird anschließend mit einer wässrigen Phase resuspendiert. Hierbei entstehen sogenannte MPV (monophasic vesicles), die mehrere Lipiddoppelschichten aufweisen. Gegenüber klassischen MLV sollen diese eine erhöhte Stabilitat in Puffer sowie erhöhte Einschlusse aufweisen.

Zu erwähnen ist letztlich auch das in der EP-A 0 349 429 beschriebene Verfahren, bei dem eine gegebenenfalls den Wirkstoff enthaltende ethanolische Lipidlosung unter leichtem Rühren in eine wässrige Phase eingetragen wird. Wie eigene Versuche zeigen, erzielt man bei diesem vorbekannten Verfahren wesentlich geringere Einschlußkapazitäten als mittels des erfindungsgemaßen Verfahrens. Zudem hat das erfindungsgemäße verfahren den Vorzug, daß mit seiner Hilfe auch bei Lipidkonzentration von über 10 % bezogen auf die ethanolische Phase hohe Einschlußkapazitäten oder sogar eine weitere Steigerung der Einschlußkapazitäten erzielen kann.

Im Vergleich zu diesen vorbekannten Methoden hat das erfindungsgemäße Verfahren den Vorzug, daß es auch in großem Maßstabe in einfacher Weise technisch durchfuhrbar ist. Die Verwendung stark toxischer oder sehr leicht entflammbarer Losungsmittel wird vermieden. Hervorzuheben ist auch die sehr gute Reproduzierbarkeit des erfindungsgemäßen Verfahrens bezüglich der erhaltenen Wirkstoffeinschlüsse und Liposomengrößen, sowie die sehr hohen Wirkstoffeinschlüsse in den Liposomen bis zu über 50 % in den Liposomen und die mittels des erfindungsgemäßen Verfahrens erhaltenen ungewöhnlich hohen Wirkstoffkonzentrationen in den Liposomensuspensionen (bis zu 800 mg/ml). Darüberhinaus ist erwahnenswert, daß es mit Hilfe des erfindungsgemäßen Verfahrens gelingt Liposomensuspensionen herzustellen, die nur einen sehr geringen Restlösungsmittelgehalt besitzen, und die eine gute Lagerfähigkeit besitzen. Das erfindungsgemäße Verfahren ist auch problemlos unter aseptischen Bedingungen durchfuhrbar.

Zur Durchführung des erfindungsgemäßen Verfahrens können die gleichen Liposomen bildende Substanzen verwendet werden wie bei den vorbekannten Verfahren.

Geeignete Liposomen bildende Substanzen sind üblicherweise amphiphatische Lipide oder Lipidgemische wie beispielsweise Phospholipide, Sphingomyeline oder Etherphospholipide. Diese Lipide können geradkettig oder verzweigte, gesättigte oder ungesättigte, gleichartige oder verschiedene Acylseitenketten tragen. Geeignete Phospholipide sind beispielsweise die Phosphatidylcholine, die Phosphatidylethanolamine, die Phosphatidylserine, die Phosphatidylinosite, die Phosphatidylglycerole oder die Phosphatidsäuren. Geeignete Etherphospholipide sind beispielsweise die Plasmalogene, (Dr. Otto-Albert Neumüller: Rompps Chemie-Lexikon; Franck'sche Verlagshandlung, Stuttgart(DE) 2665, 3159, 3920 und 4045).

Zur Herstellung der Liposomen kann man auch Gemische dieser Lipide und insbesondere auch Gemische dieser Lipide mit Cholesterol Cholesterolhemisuccinat, α-Tocopherol, α-Tocopherolhemisuccinat und/oder Ladungsträgern wie zum Beispiel Stearylamin, Stearinsäure, Diethylphosphat, Olsäure, Palmitinsäure, Gallensäure wie zum Beispiel Cholsäure, Glykocholsäure verwenden. Geeignete Gemische können etwa bis zu 60 Molprozent Cholesterin und bis zu 20 Molprozent Ladungsträger enthalten.. Als Losungsmittel für die Phospholipide oder Gemische verwendet man vorzugsweise Isopropanol oder insbesondere Ethanol.

Grundsätzlich sollte das erfindungsgemäße Verfahren auch mit anderen wasserlöslichen niedrig siedenden Losungsmitteln als niederen Alkoholen durchführbar sein; geeignet ist beispielsweise Tetrahydrofuran. Die Durchführung des Verfahrens ist aber wesentlich aufwendiger als das erfindungsgemäße Verfahren.

Zur Durchführung des erfindungsgemäßen Verfahrens werden vorzugsweise alkoholische Losungen verwendet, die 0,2 g bis 30 g Liposomenbildende(s) Substanz(gemisch) pro 100 ml Losungsmittel enthalten. Erforderlichenfalls werden diese Lösungen durch Erwärmen der Komponenten dargestellt.

Das erfindungsgemäße Verfahren wird, wie bereits erwähnt wurde in der Weise durchgeführt, daß man die wässrige Phase unter Vermischen (wie zum Beispiel kräftigem Rühren) in die alkoholische Lösung eintragt und den niederen Alkohol beispielsweise mittels Vakuumdestillation oder durch Einleiten von Stickstoff entfernt.

Gegebenenfalls ist die nach der Vereinigung erhaltene Dispersion einige Zeit (ca. 10 bis 180 Minuten) bei einer Temperatur von etwa 20 bis 90° C zu mischen, ehe man das Lösungsmittel ganz oder teilweise abtrennt.

Die für das erfindungsgemäße Verfahren geeigneten Temperaturen sind von der Löslichkeit der Liposomenbildenden Substanzen sowie deren Phasenübergangstemperatur (tc), der Hitzestabilität des Wirkstoffs oder Wirkstoffgemisches und dem Dampfdruck des abzutrennenden Alkohols abhangig, sie liegt vorzugsweise zwischen 20° C und 90° C und insbesondere zwischen 40° C und 70° C. Im allgemeinen wird es ausreichend sein, mit einem Vakuum von 10 h Pa bis 100 h Pa zu arbeiten.

Die für das erfindungsgemäße Verfahren verwendete wässrige Phase kann gewünschtenfalls Puffersubstanzen und/oder isotonisierende Zusätze enthalten. Geeignete Zusätze sind beispielsweise anorganische oder organische Salze oder Puffersubstanzen, wie Natriumchlorid, TRIS-Puffer, Phosphat-Puffer, Citrat-Puffer, Glycin-Puffer, Citrat-Phosphat-Puffer, Maleat-Puffer, etc. Mono- oder Disaccharide, wie Glucose, Lactose, Saccharose oder Trehalose, Zuckeralkohole, wie Mannit, Sorbit, Xylit oder Glycerin oder wasserlösliche Polymere, wie Dextran oder Polyethylenglykol.

Da die Lipide und auch einige Wirkstoffe oxidationsempfindlich sind, kann die wässrige Phase mit Antioxidantien, wie Natriumascorbat, Tocopherol oder Natriumhydrogensulfit versetzt werden.

Die nach dem erfindungsgemäßen Verfahren hergestellten wässrigen Liposomensuspensionen dienen vorzugsweise zur Verkapselung wasserlöslicher Wirkstoffe.

Solche wasserlöslichen Wirkstoffe sind beispielsweise Diagnostika, wie die Röntgenkontrastmittel Iotrolan, Iopromid, Iohexol, Iosimid, Metrizamid, Salze von Amidoessigsäure, Iotroxinsäure, Iopamidol, 5-Hydroxyacetamido-2,4,6-triiod-isophtalsäure-(2,3-dihydroxy-N-methylpropyl)-(2-hydroxyethyl)-diamid (= ZK 119095) und 3-Carbamoyl-5-[N-(2-hydroxyethyl)-acetamido]-2,4,6-triiod-benzoesäure-[(1RS,2SR)-2,3-dihydroxy-1-hydroxymethylpropyl]-amid (= ZK 139129) oder NMR-Kontrastmittel, wie das Gadolinium-DTPA, das Gadolinium-DOTA und den Gadoliniumkomplex des 10-[1-Hydroxymethyl-2,3-dihydroxypropyl]-1,4,7-tris-(carboxymethyl)-1,4,7,10-tetraazacyclodecans].

Geeignete therapeutische Wirkstoffe sind unter anderen Antibiotika, wie Gentamycin oder Kanamycin, Cytostatica, wie Doxorubicin-Hydrochlorid oder Cyclophosphamid und Virustatica, wie Vidarabin oder Wirkstoffe, wie das Mitoxantron-Hydrochlorid.

Diese wasserlöslichen Wirkstoffe werden vor Durchführung des erfindungsgemäßen Verfahren in der wässrigen Phase gelöst.

Darüberhinaus können die wässrigen Liposomensuspensionen auch zur Verkapselung von in Wasser schwer löslichen Wirkstoffe verwendet werden.

Solche Wirkstoffe sind beispielsweise Pflanzenschutzmittel, wie schwer lösliche Insektizide oder Herbizide und insbesondere schwer lösliche pharmazeutische Wirkstoffe.

In Wasser schwer lösliche oder unlösliche pharmazeutische Wirkstoffe folgender Wirkstoffgruppen eignen sich beispielsweise zur Herstellung wässriger Suspensionen nach dem erfindungsgemäßen Verfahren.

Gestagen wirksame Steroidhormone wie beispielsweise das 13-Ethyl-17β-hydroxy-18,19-dinor-17α-pregn-4-en-20yl-3-on (=Levonorgestrel), das 13-Ethyl-17β-hydroxy-18,19-dinor-17α-pregna-4,15-dien-20yn-3-on (=Gestoden) oder das 13-Ethyl17β-hydroxy-11-methylen-18,19-dinor-17α-pregn-4-en-20yn

(Desorgestrel), Estrogen wirksame Steroidhormone wie 3-Hydroxy-1,3,5(10)-estratrien-17-on (=Ostron) oder 1,9-Nor-17α-pregna-1,3,5(10)-trien-20-yn-3,17β-diol (Ethinylostradiol).

Androgen wirksame Steroidhormone wie zum Beispiel 17β-Hydroxy-4-androsten-3-on (=Testosteron) und dessen Ester oder 17β-Hydroxy-1α-methyl-5α-androsten-3-on (=Mesterolon).

Antiandrogen wirksame Steroidhormone wie zum Beispiel das 17α-Acetoxy-6-chlor-1β,2β-dihydro-3 H-cyclopropa[1,2]-pregna-1,4,6-trien-3,20-dion (Cypoteronacetat).

Kortikoide wie zum Beispiel das 11β,17α,21-Trihydroxy-4-pregnen-3,20-dion (=Hydrocortison), das 11β,17α,21-Trihydroxy-1,4-pregnadien-3,20-dion (=Prednisolon), das 11β,17α-21-Trihydroxy-6α-methyl-1,4-pregnatrien-3,20-dion (=Methylprednisolon) und das 6α-Fluor-11β,21-dihydroxy-16α-methyl-1,4-pregnadien-3,20-dion (=Difluocortolon) und deren Ester.

Ergoline wie zum Beispiel der 3-(9,10-Dihydro-6-methyl-8α-ergolinyl)-1,1-diethylharnstoff (=Ergolin), der 3-(2-Brom-9,10-dihydro-6-methyl-8α-ergolinyl)-1,1-diethylharnstoff (=Bromergolin) oder der 3-(6-Methyl-8α-ergolinyl)-1,1-diethylharnstoff (=Tergurid).

Antihypertonika wie zum Beispiel das 7α-Acetylthio-17α-hydroxy-3-oxo-4-pregnen-21-carbonsäure-γ-lacton (=Spironolacton) oder das 7α-Acetylthio-15β,16β-methylen-3-oxo17α-pregna-1,4-dien-21,17-carbolacton (=Mespirenon).

Antikoagulantia, wie zum Beispiel die 5-[Hexahydro-5-hydroxy-4-(3-hydroxy-4-methyl-1-octen-6-ynyl)-2(1H)-pentalenyliden)]-pentansäure (=Iloprost).

Psychopharmaka, wie zum Beispiel das 4-(3-Cyclopentyloxy-4-methoxy-phenyl-2-pyrrolidon (=Rolipram) und das 7-Chlor-1,3-dihydro-1-methyl-5-phenyl-2H-1,4-benzodiazepin-2-on (=Diazepam).

Carotinoide, wie zum Beispiel das α-Carotin und das β-Carotin.

Fettlösliche Vitamine, wie zum Beispiel Vitamine der Vitamin A-, Vitamin D-, Vitamin E-und Vitamin K-Gruppe.

Eine weitere Gruppe sind β-Carboline, wie sie beispielsweise in den Europäischen Patentanmeldungen 234,173 und 239,667 beschrieben sind. Als β-Carboline seien beispielsweise genannte der 6-Benzoyloxy-4-methoxymethyl-βcarbolin-3-carbonsäure-isopropylester (=Becarnil) und der 5-(4-Chlorphenoxy)4-methoxymethyl-β-carbolin-3-carbonsäure-isopropylester (=Cl-PHOCIP).

Erwähnenswert sind auch schwerlösliche oder unlösliche Kontrastmittel, wie das Röntgenkontrastmittel Iodipamidethylester oder NMR-Kontrastmittel wie die Eisen- oder Manganporphyrinchelate oder auch die Magnetite.

Als geeignete Wirkstoffe seien ferner die Salizylsäure, die Retinolsäure und die Azelainsäure erwähnt. Des weiteren können Antimycotika wie das Amphotericin-B, Econazol oder Meconazol verwendet werden.

Diese Wirkstoffe werden vor Durchführung des erfindungsgemäßen Verfahrens in der alkoholischen Lösung gelöst oder in ihr und/oder der wässrigen Phase suspendiert.

Bei dem erfindungsgemäßen Verfahren werden pro g Liposomen bildender Substanz(mischung) im allgemeinen 0,05 bis 10 g und vorzugsweise 1 bis 3 g Wirkstoff eingesetzt. Mittels des erfindungsgemäßen Verfahrens kann man in einfacher Weise sterile Liposomensuspensionen herstellen indem man beide Lösungen vor der Durchfuhrung des Verfahrens sterilfiltriert und alle anschließenden Prozeßschritte unter aseptischen Bedingungen durchfuhrt.

Die in den Liposomensuspensionen erzielbaren Einschlusse sind naturgemäß von der Art des Wirkstoff und der (des) Liposomen bildenden Substanz(gemisches) sowie deren Verhältnis zueinander abhängig. Gewünschtenfalls kann der unverkapselte Wirkstoff beispielsweise mittels Ultrafiltration, Dialyse, Mikrofiltration oder Zentrifugieren entfernt werden.

Die erhaltene Liposomensuspension kann direkt aufbewahrt werden. Alternativ kann die Liposomensuspension durch Lyophilisation in eine stabile Lagerform überführt werden. Vor der Lyophilisation kennen dem zu lyophilisierenden Gut, wenn notig, unter anderem beispielsweise Zusätze wie Mannitol oder Sorbitol und/oder Elektrolyte und viskositätsbeeinflussende Agentien wie Natriumchlorid zugesetzt werden.

Die Lyophilisate können mit bidestilliertem Wasser oder wässrigen Phasen, welche die gleichen Zusätze wie die zur Liposomenherstellung verwendeten wässrigen Phasen enthalten können, resuspendiert werden.

Die Menge des Resuspensionsmediums kann dabei 0.5 bis 20, vorzugsweise jedoch 1 bis 6 ml pro g Lyophilisat betragen. Die erhaltenen Suspensionen können direkt oder nach Filtration über Filter geeigneter Porengröße verwendet werden.

Die mittels des erfindungsgemäßen Verfahrens hergestellten Liposomen können beispielsweise wenn sie Kontrastmittel für NMR oder Röntgendiagnostik enthalten bei verschiedenen diagnostischen Verfahren eingesetzt werden. Hierzu zählen beispielsweise die Tumordiagnostik in Organen des retikuloendothelialen Systems (zum Beispiel Leber und Milz) oder anderen intravasal zugänglichen Geweben sowie auch die Arthrographie. Die extravasale Applikation solcher Liposomensuspensionen (wie zum Beispiel subcutan, intramusculär oder intraperitoneal) kann ebenfalls für diagnostische Zwecke wie etwa die indirekte und direkte Lymphographie oder therapeutische Zwecke (wie im. Depotpräparate) verwendet werden. Selbstverständlich ist es auch möglich die Zubereitungen oral zu applizieren, wobei man die Präparate gegebenenfalls in Magensaft resistenten Kapseln abfüllen kann.

Ferner können mittels des erfindungsgemäßen Verfahrens hergestellte wirkstoffhaltige Liposomensuspensionen bei geeigneter Lipidzusammensetzung auch als blood pool agents oder als zielgerichtete Arzneistoffträger angewendet werden.

Die nachfolgenden Ausführungsbeispiele dienen zur näheren Erläuterung dar Erfindung. In diesen Beispielen werden folgende Abkürzungen verwendet:
- PC: = Phophatidylcholin S 100 der Firma Lipoid KG
- CH: = Cholesterol gepulvert (JP) der Firma Merck AG
- SS: = Stearinsäure puriss der Firma Fluka AG
- DPPG: = Dipalmitoylphosphatidylglycerol DPPG der Firma Lipoid KG
- HSPC: = hydriertes Sojalecitin (Phospholipon 90H) der Firma Nattermann AG
- DCP: = Dicetylphosphat der Firma Sigma

### Beispiel 1

9.25 g Lipidgemisch (PC/CH/SS - 4:5:1 mol:mol:mol) werden bei 70° C in 100 ml ml Ethanol gelost und sterilfiltriert. Dann überführt man die Losung in ein auf 55° C temperiertes Reaktionsgefäß und mischt diese unter Rühren (300 Umdrehungen pro Minute) mit einer sterilfiltrierten Wirkstofflösung (enthaltend 27,75 g Iopromid ad 200 ml wässrigem 20 mM Tris-HCl-Puffer; pH 7,5).

Anschließend destilliert man den Alkohol bei 55° C im Vakuum von 5000 Pa ab und untersucht die entstandene Liposomensuspension auf ihre Eigenschaften.

Dann wird die Liposomensuspension direkt in Portionen von 20 g in 50 ml Glasinfusionsflaschen abgefüllt und diese lyophilisiert. Die erhaltenen Lyophilisate werden dergestalt resuspendiert, daß eine Wirkstoffkonzentration von ca. 200 mg/ml entsteht und ebenfalls untersucht.

### Beispiel 2

Die Durchfuhrung des Versuchs erfolgt wie in Beispiel 1 beschrieben, jedoch werden 18,5 g Iopromid verwendet.

### Beispiel 3

Die Durchführung des Versuches erfolgt wie in Beispiel 2 beschrieben, jedoch werden 13,9 g Lipidgemisch verwendet.

### Beispiel 4

Die Durchführung des Versuches erfolgt wie in Beispiel 2 beschrieben, jedoch wrden 18,5 g Lipidgemisch verwendet.

### Beispiel 5

Die Durchführung des Versuches erfolgte wie in Beispiel 2 beschrieben, jedoch wird Iotrolan anstelle von Iopromid verwendet.

### Beispiel 6

Die Durchführung des Versuches erfolgt wie in Beispiel 2 beschrieben, jedoch wird Iopamidol anstelle von Iopromid verwendet.

### Beispiel 7

Die Durchführung des Versuches erfolgt wie in Beispiel 2 beschrieben, jedoch wird Iohexol anstelle von Iopromid verwendet.

### Beispiel 8

Die Durchführung des Versuches erfolgt wie in Beispiel 2 beschrieben, jedoch wird das nichionische Kontrastmittel 5-Hydroxyacetamid-2,4,6-triiod-isophthalsäure-(2,3-dihydroxy-N-methylpropyl)-(2-hydroxyethyl)-diamid anstelle von Iopromid verwendet.

### Beispiel 9

Die Durchführung des Versuches erfolgte wie in Beispiel 2 beschrieben, jedoch wird ein Lipidgemisch PC/CH/SS - 4:4:2 mol:mol:mol eingesetzt.

### Beispiel 10

Die Durchführung des Versuches erfolgt wie in Beispiel 2 beschrieben, jedoch wird ein Lipidgemisch PC/CH/PG 4:5:1 mol:mol:mol eingesetzt.

### Beispiel 11

Die Durchführung des Versuches erfolgt wie in Beispiel 2 beschrieben, jedoch wird ein Lipidgemisch PC/CH/SS 6:3:1 mol:mol:mol eingesetzt.

### Beispiel 12

Die Durchführung des Versuches erfolgt wie in Beispiel 2 beschrieben, jedoch wird ein Lipidgemisch PC/CH/DCP 4:5:1 eingesetzt.

### Beispiel 13

Die Durchführung des Versuches erfolgt wie in Beispiel 2 beschrieben, jedoch wird ein Lipidgemisch PC/CH 1:1 eingesetzt.

### Beispiel 14

Die Durchführung des Versuches erfolgt wie in Beispiel 2 beschrieben, jedoch wird ein Lipidgemisch PC/HSPC/CH/SS 2:2:5:1 eingesetzt.

### Beispiel 15

Die Durchführung des Versuches erfolgt wie in Beispiel 2 beschrieben, jedoch wurde die Ansatzgröße verzehnfacht.

### Beispiel 16

Die Durchführung des Versuches erfolgt wie in Beispiel 2 beschrieben, jedoch in einem einhundertfünfzigfach größeren Ansatz.

Die nachfolgende Tabelle zeigt die in den Beispielen 1 bis 16 erzielten Ergebnisse.

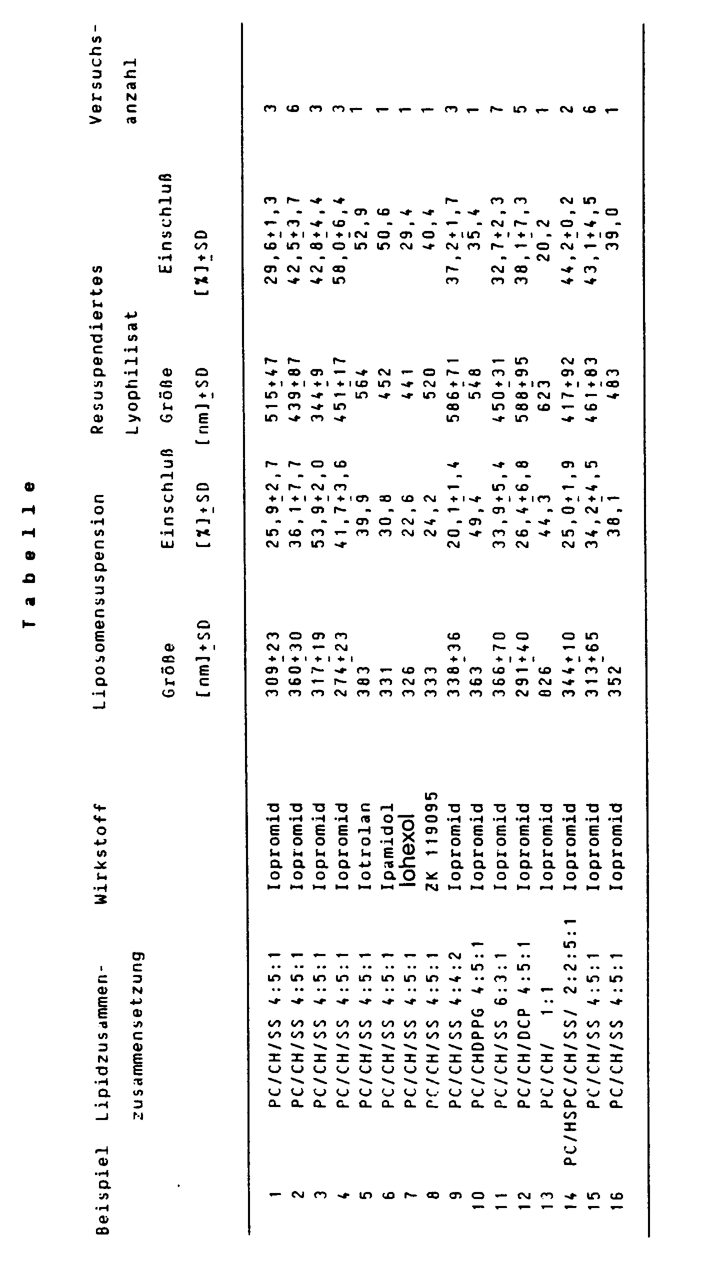

### Beispiel 17

Der Restethanolgehalt von gemäß Beispiel 2 hergestellten Liposomensuspensionen vor und nach Lyophilisation wird an 8 Chargen mittels Gaschromatographie bestimmt. In der ursprünglichen Liposomensuspension werden die Ethanolgehalte mit 0,59 ± 0,010 % (m/V) bestimmt.

### Beispiel 18

Gemäß Beispiel 2 hergestellte Liposomensuspensinnen und Lyophilisate werden bei 4, 25 und 40° C eingelagert. Zur Beurteilung der Stabilität werden Aussehen, Größe Einschluß und pH-Wert -im Falle der Lyophilisate nach Resuspension - zum jeweiligen Zeitpunkt bestimmt.

Nach mehrmonatiger Lagerung zeigten die resuspendierten Lyophilisate hinsichtlich der untersuchten Größen keine signifikanten Abweichungen.

Die so hergestellten Liposomensuspensionen zeigen in pharmakologischen Tests folgende Eigenschaften:

### Test A

Gemäß Beispiel 2 hergestellte resuspendierte Lyophilisate werden hinsichtlich ihrer akuten Toxizität nach einmaliger Applikation an Maus und Ratte untersucht.
Die jeweilige LD50 wird zu:
2,8 g I/kg KGW Maus (Gesamtiod( bzw.
3,0 g I/Kg KGW Ratte
bestimmt.

### Test B

Für gemäß Beispiel 2 hergestellte resuspendierte Lyophilisate wird die subkakute Toxizität an Ratten (n = 6) bestimmt.

Nach 6-maliger Gabe von jeweils 1 g I/kg KGW (Gesamtiod) im Abstand von jeweils 3 Tagen werden keine Veränderungen histo-pathologischer bzw. klinischer Parameter festgestellt.

### Test C

Gemäß Beispiel 2 erhaltene resuspendierte Lyophilisate werden hinsichtlich ihrer Organverteilung nach i.v. Gabe an Ratten untersucht.

1 Stunde nach Gabe von 100 mg I/kg KGW (Gesamtiod) werden in der Leber 0,54 mg I/g Feuchtgewicht entsprechend 24 % der applizierten Dosis nachgewiesen.

### Test D

Gemäß Beispiel 2 erhaltene resuspendierte Lyophilisate werden Lebertumortragenden Kaninchen mit einer Dosis von 100 mg Gesamtiod/kg KGW i.v. appliziert. Der sich ergebende Dichteunterschied Leber/Tumor beträgt nach einer Stunde 35 Hounsfied units (HU).

### Test E

Gemäß Beispiel 2 erhaltene resuspendierte Lyophilisate werden hinsichtlich ihrer Eignung zur indirekten CT-Lymphographie am Hund untersucht.

3 Stunden nach interdigitaler Gabe von 200 mg Gesamtiod (in 3 ml Liposomensuspension) werden am CT maximal 200 HU Dichteanhebung in den poplietalen und iliakalen Lymphknoten gemessen.

## Patentansprüche

1. Verfahren zur Herstellung von wirkstoffhaltigen wäßrigen Liposomensuspensionen aus einer Lösung einer (eines) Liposomen bildenden Substanz(gemisches) in einem niederen Alkohol mit maximal 3 Kohlenstoffatomen und einer wässrigen Phase wobei der Wirkstoff oder das Wirkstoffgemisch in der Lipidlösung und/oder der wäßrigen Phase gelöst oder suspendiert ist, dadurch gekennzeichnet, daß man die wäßrige Phase unter Vermischen in die alkoholische Lösung einträgt, den niederen Alkohol mittels Vakuumdestillation oder durch Einleiten von Stickstoff entfernt und gegebenenfalls die Liposomensuspension lyophilisiert.

2. Verfahren zur Herstellung von wirkstoffhaltigen wäßrigen Liposomensuspensionen gemäß Patentanspruch 1, dadurch gekennzeichnet, daß der Wirkstoff in Wasser gut löslich ist und sich in der wäßrigen Phase befindet.

3. Verfahren zur Herstellung von wirkstoffhaltigen wäßrigen Liposomensuspensionen gemäß Patentanspruch 1 und 2, dadurch gekennzeichnet, daß der Wirkstoff ein Kontrastmittel ist.

4. Verwendung von gemäß Verfahren nach Anspruch 1 bis 3 hergestellten Liposomensuspensionen zur Herstellung von Mitteln für die Tumordiagnostik.

5. Verwendung von gemäß Verfahren nach Anspruch 1 bis 3 hergestellten Liposomensuspensionen zur Herstellung von Mitteln für die indirekte Lymphographie.

## Claims

1. Process for the preparation of active-ingredient-containing aqueous liposome suspensions from a solution of a liposome-forming substance (or substance mixture) in a lower alcohol having a maximum of 3 carbon atoms and an aqueous phase, the active ingredient or active ingredient mixture being dissolved or suspended in the lipid solution and/or the aqueous phase, characterised in that the aqueous phase is introduced, with mixing, into the alcoholic solution, the lower alcohol is removed by means of vacuum distillation or by the introduction of nitrogen and, optionally, the liposome suspension is lyophilised.

2. Process for the preparation of active-ingredient-containing aqueous liposome suspensions according to patent claim 1, characterised in that the active ingredient is readily soluble in water and is present in the aqueous phase.

3. Process for the preparation of active-ingredient-containing aqueous liposome suspensions according to patent claims 1 and 2, characterised in that the active ingredient is a contrast medium.

4. Use of liposome suspensions prepared in accordance with the process according to claims 1 to 3 for the preparation of media for tumour diagnostics.

5. Use of liposome suspensions prepared in accordance with the process according to claims 1 to 3 for the preparation of media for indirect lymphography.

## Revendications

1. Procédé pour la préparation de suspensions de liposomes aqueuses contenant des principes actifs à partir d'une solution d'une substance (mélange de substances) formant des liposomes dans un alcool inférieur avec au maximum trois atomes de carbone et d'une phase aqueuse, le principe actif ou le mélange de principes actifs étant mis en suspension ou dissout dans la solution lipidique et/ou dans la phase aqueuse, caractérisé en ce qu'on introduit la phase aqueuse, en agitant, dans la solution alcoolée, on sépare l'alcool inférieur par distillation sous vide ou par admission d'azote et on lyophilise éventuellement la suspension de liposomes.

2. Procédé pour la préparation de suspensions de liposomes aqueuses contenant des principes actifs selon la revendication 1, caractérisé en ce que le principe actif a une bonne solubilité dans l'eau et se trouve dans la phase aqueuse.

3. Procédé pour la préparation de suspensions de liposomes aqueuses contenant des principes actifs selon la revendication 1 et 2, caractérisé en ce que le principe actif est un produit de contraste.

4. Utilisation de suspensions de liposomes préparées selon le procédé selon la revendication 1 a 3, pour la préparation d'agents pour les diagnostics de tumeurs.

5. Utilisation de suspensions de liposomes préparées selon le procédé selon la revendication 1 à 3, pour la préparation d'agents pour la lymphographie indirecte.
